# EUROPEAN PATENT APPLICATION

(11) **EP 3 443 956 A1**
(43) Date of publication of application: **20.02.2019**
(21) Application number: 17306080.7
(22) Date of filing: 18.08.2017
(51) Int. Cl.: A61K 31/165, A61K 31/352, A61K 31/4418, A61P 25/24, A61P 25/22, A61P 25/18, A61P 25/30

(54) **A COMPOUND INCREASING ENDOCANNABINOIDS CONCENTRATION IN A SUBJECT FOR USE FOR PREVENTING AND/OR TREATING A SOCIAL BEHAVIOR DISORDER**

(71) Applicant: INSTITUT NATIONAL DE LA SANTE ET DE LA RECHERCHE MEDICALE (INSERM), 75013 Paris (FR); Université d'Aix-Marseille, 13007 Marseille (FR)
(72) Inventor: The designation of the inventor has not yet been filed
(74) Representative: Lavoix

(57) **Abstract**

The present invention concerns a compound increasing endocannabinoids (eCBs) concentration, a CB1 R agonist or a TRPV1 agonist for use for preventing and/or treating a social behaviors disorder, such as autism, excessive shyness, sociopathy, anxiety, mood disorder or depression. Indeed, inventors show a sex specific modulation of social interaction and medial prefrontal cortex endocannabinoid/mGlu5-dependent LTD (a critical actor of plasticity and reliable prefrontal endophenotypes of neuropsychiatric diseases) in adult rats. TRPV1 blocking leads to reduced social interaction and blocked endocannabinoid/mGlu5-dependent LTD in medial prefrontal cortex(mPFC) of female rats whereas CB1 R blocking leads to blocked endocannabinoid/mGlu5-dependent LTD in mPFC of adult male. *In utero* exposure to cannabinoids modified social behaviors as well as mPFC endocannabinoid/mGlu5-dependent LTD at adulthood of male rats but not female rats. Increasing endocannabinoids concentration could compensate the effects of *in utero* exposure in males through activation of CB1 R.

## Description

The present invention concerns a compound increasing endocannabinoids (eCBs) concentration, a CB1 R agonist, or a TRPV1 agonist for use for preventing and/or treating a social behavior disorder, such as autism, excessive shyness, sociopathy, anxiety, mood disorder or depression.

### BACKGROUND TO THE INVENTION

To date, our understanding of the relative contribution and potential overlapping roles of the endocannabinoids in the regulation of brain function and behavior is still limited.

The endocannabinoid system (ECS) is involved in a variety of physiological processes including appetite, pain-sensation, mood, and memory, and in mediating the psychoactive effects of cannabis.

Cannabinoid receptor type 1 (CB1 R) has been identified as a primary endocannabinoid receptors. CB1 receptors are found predominantly in the brain and nervous system, as well as in peripheral organs and tissues, and are the main molecular target of the endocannabinoid ligand anandamide. 2-Arachidonoylglycerol (2-AG) is another endocannabinoid that acts as a full agonist at the CB1 receptor and is involved in the regulation of appetite, immune system functions and pain management.

Anandamide and 2 A-G are also known to bind to the transient receptor potential cation channel subfamily V member 1 (TRPV1), also known as the capsaicin receptor or the vanilloid receptor 1. TRPV1 receptors are found mainly in the nociceptive neurons of the peripheral nervous system, but are also present in many other tissues, including the central nervous system. TRPV1 is involved in the transmission and modulation of pain, as well as the integration of diverse painful stimuli.

The inventors determined that *in utero* exposition to cannabinoids in rat sex-specifically modified social behaviors at adulthood. In particular adult male rats which had been exposed *in utero* spent less time in social interactions and had less contact with their congeners, whereas female rats which had been exposed *in utero* showed a normal behavioral profile. This modification in male behavior was accompanied with deletion of the mPFC (medial prefrontal cortex) eCB/mGlu5-dependent LTD (endocannabinoid mediated/mGlu5-dependent long-term depression) in adult male rats exposed *in utero,* but there was no modification of the eCB-LTD in the accumbens. Social interaction behavior is modulated in the prefrontal cortex, and endocannabinoid/mGlu5-dependent LTD is a critical actor of plasticity and reliable prefrontal endophenotypes. eCB-mediated plasticity in the accumbens is instrumental in underlying depression-like and emotional behavior. Interestingly, no modification was observed in adult female rats mPFC eCB-LTD nor accumbens eCB-LTD. These data show that male *in utero* exposure to cannabinoids decreases social interactions and selectively abolishes the development of mGlu5-eCB-mediated synaptic plasticity in the adult rat PFC but not in accumbens, thus showing no effect on cognition.

Using a rat model, the inventors showed that eCB-LTD of excitatory mPFC synapses was mediated by CB1 receptors in male rats, and by TRPV1 receptors in female rats.

The inventors studied the effect of the increase of anandamide by URB597 (an inhibitor of FAAH, anandamide's main degrading enzyme) on adult male rats exposed *in utero* to cannabinoids and showed restoration of the mPFC eCB/LTD and normalization of social behaviors. The inventors showed that these effects were mediated by CB1 R activation and not by TRPV1 activation.

The inventors also studied the effect of an TRPV1 antagonist (capsazepine) on social interaction of female rats and showed a reduction in frequency and duration of social interaction, confirming the involvement of TRPV1 rather than CB1 receptors in the modulation of social behavior at adulthood in female rats. These effects were also replicated in adult female rats exposed to cannabinoids *in utero.*

These results thus show that TRPV1 blocking leads to reduced social interaction and blocked endocannabinoid/mGlu5-dependent LTD in medial prefrontal cortex (mPFC) of female rats, whereas CB1 R blocking leads to blocked endocannabinoid/mGlu5-dependent LTD in mPFC of adult male.

Altogether, in light of the relationship between endocannabinoids and social interactions identified by the inventors, these results demonstrate that a compound increasing endocannabinoids concentration in a subject, by activating CB1 or TRPV1 receptor, can be used for the prevention or treatment of a social behaviors disorder.

### BRIEF SUMMARY OF THE INVENTION

The invention concerns a compound increasing endocannabinoids concentration in a subject for use for preventing and/or treating a social behavior disorder.

The invention further concerns a CB1 R agonist for use for preventing and/or treating a social behavior disorder social in a male subject.

The invention also concerns a TRPV1 agonist for use for preventing and/or treating a social behavior disorder social in a female subject.

### DETAILED DESCRIPTION OF THE INVENTION

The invention concerns a compound increasing endocannabinoids concentration in a subject for use for preventing and/or treating a social behavior disorder.

The invention concerns the use of a compound increasing endocannabinoids concentration for the manufacture of a medicament for preventing and/or treating a social behavior disorder in a subject.

The invention also concerns a method for preventing and/or treating a social behavior disorder comprising administering a therapeutically effective amount of a compound increasing endocannabinoids concentration to a subject in need thereof.

As used herein, the term "a **compound increasing endocannabinoid concentration"** refers to compound that permit the increase of at least one endocannabinoid, or all endocannabinoids, by increasing its synthesis and/or limiting its degradation and/or increasing its half-life *in vivo.* In particular said compound inhibits a degrading enzyme of endocannabinoids, and preferably inhibits the fatty acid amide hydrolase (FAAH). Advantageously the compound increasing endocannabinoid concentration increases the concentration of anandamide and/or 2-Arachidonoylglycerol (2-AG).

The **"subject"** or **"individual"** may be, for example, a human or non human mammal, such as a rodent (mouse, rat), a feline, a canine or a primate, affected by or likely to be affected by a social behaviors disorder. Typically, the subject is a human. Preferably the subject is an adult.

Preferably the compound increasing endocannabinoid concentration permits the increasing of endocannabinoids concentration in the medial prefrontal cortex (mPFC) of the subject.

In the context of the invention, the term **"treating"** or **"treatment",** refers to a therapeutic use (i.e. on a subject having a given disease) and means reversing, alleviating, inhibiting the progress of one or more symptoms of such disorder or condition. Therefore, treatment does not only refer to a treatment that leads to a complete cure of the disease, but also to treatments that slow down the progression of the disease and/or prolong the survival of the subject.

By **"preventing"** is meant a prophylactic use (i.e. on a subject susceptible of developing a given disease).

By **"a social behavior disorder"** is meant any disorder which has deleterious effect on social behavior, such as autism, excessive shyness, sociopathy, schizophrenia, bipolar disorder, irritability, addiction tendencies, anxiety, mood disorder or depression such as major depressive illness. Such disorders also include social anxiety disorder (SAD) and avoidant personality disorder. Preferably social behaviors disorder according to the invention is autism, excessive shyness, sociopathy, schizophrenia, bipolar disorder, irritability, addiction tendencies, or depression.

The invention further concerns a CB1 R agonist for use for preventing and/or treating a social behavior disorder social in a male subject. The invention also concerns a TRPV1 agonist for use for preventing and/or treating a social behavior disorder social in a female subject.

The invention concerns the use of a CB1 R agonist for the manufacture of a medicament for preventing and/or treating a social behavior disorder in a male subject. The invention concerns the use of a TRPV1 agonist for the manufacture of a medicament for preventing and/or treating a social behavior disorder in a female subject.

The invention also concerns a method for preventing and/or treating a social behavior disorder comprising administering to a male subject in need thereof a therapeutically effective amount of a a CB1 R agonist.

The invention concerns a method for preventing and/or treating a social behavior disorder comprising administering to a female subject in need thereof a therapeutically effective amount of a TRPV1 agonist.

The cannabinoid receptor type 1, often abbreviated as CB1 R, is a G protein-coupled cannabinoid receptor located in the central and peripheral nervous system. It is activated by the endocannabinoid neurotransmitters such as anandamide and 2-arachidonoylglycerol (2-AG); by plant cannabinoids, such as the compound Delta9-Tetrahydrocannabinol (THC), and by synthetic analogues of THC.

The transient receptor potential cation channel subfamily V member 1 (TRPV1), also known as the capsaicin receptor and the vanilloid receptor 1, is a nonselective cation channel. TRPV1 receptors are found mainly in the nociceptive neurons of the peripheral nervous system, but they have also been described in many other tissues, including the central nervous system. TRPV1 is involved in the transmission and modulation of pain (nociception), as well as the integration of diverse painful stimuli.

As used herein, the term **"receptor agonist"** refers to any molecule that induces, increases or stimulates said receptor activity. The biological activity of a receptor depends on the amount of ligand (i.e. its expression level) as well as on the activity of the ligand. Therefore, the agonist may increase the expression of said ligand or increase the activity of said ligand on the receptor. Preferably the receptor agonist is a positive allosteric modulator. A positive allosteric modulator (PAM) or allosteric enhancer induces an amplification of the orthosteric agonist's effect, either by enhancing the binding affinity or the functional efficacy of the orthosteric agonist for the target protein

**"CB1R agonist"** refers to a molecule that partially or fully induces, increases or stimulates biological activities of CB1 R. This includes, but is not limited to, the increasing of the expression or activity of said CB1 R ligand. In an embodiment of the invention, the CB1 R is a cannabinoid, or pharmaceutically acceptable salts thereof. CB1 R agonists include for instance Dronabinol, anandamide, Epigallocatechin, Epicatechin, Kavain, Yangonin, N-Arachidonoyl dopamine, Cannabinol, HU-210, 11-Hydroxy-THC, Levonantradol, 2-Arachidonyl glyceryl ether, JWH-073, Tetrahydrocannabinol, 2-Arachidonoylglycerol, AM-2201, CP 55,940, JWH-018, WIN 55,212-2 or GAT228 or pharmaceutically acceptable salts thereof. Preferably the CB1R agonist is a positive allosteric modulator such as lipoxin A4, GAT211, GAT 228, GAT 229, ZCZ011, RTI-370, RTI-371, JHW007 and analogues thereof (Scott et al. 2017).

**"TRPV1 agonist"** refers to a molecule that partially or fully induces, increases or stimulates biological activities of TRPV1. This includes, but is not limited to, the increasing of the expression or activity of said TRPV1 ligand. TRPV1 agonists include for instance a lipid vanilloid, or pharmaceutically acceptable salts thereof. Suitable agonist molecules specifically include, but are not limited to biological molecules such as a lipid, protein, polypeptide, peptide, or non-biological large or small molecules (less than 10 kDa), in particular small organic molecules. Preferably the TRPV1 agonist is a positive allosteric modulator such as MRS1477 and MRS1477 analogues (Lebovitz et al. 2012).

**Methods for determining whether a compound is an agonist** are well-known by the person skilled in the art. For example, the person skilled in the art can assess whether a compound induces the receptor expression. According to the invention, the **"level of expression of the receptor"** is determined by detecting a nucleic acid comprising the sequence of said receptor, a variant, a fragment, a complementary sequence or a corresponding RNA sequence thereof.

**Other methods for determining whether a compound is an agonist** may be for example, by measuring the biological activity of the receptor, through measuring one of the phenomenon in which receptor is known to play a role. For instance, the inventors have demonstrated that CB1 R is implicated in mPFC eCB-LTD response in male. Indeed, the biological activity of CB1 R and notably the increased of CB1 R biological activity, may be assessed through measuring the mPFC eCB-LTD response in adult male rats exposed *in utero* to cannabinoids.

**"Positive allosteric modulator"** refers to an agonist that has little or no intrinsic activity but enhances receptor activation by an orthosteric agonist and, as a result, would act conditionally to potentiate only the receptor when the orthosteric agonist is present. Positive allosteric modulators potentiate binding and/or signalling by an orthosteric agonist.

**"Pharmaceutically"** or **"pharmaceutically acceptable"** refers to molecular entities and compositions that do not produce an adverse, allergic or other untoward reaction when administered to a mammal, especially a human, as appropriate. A pharmaceutically acceptable carrier, excipient or diluent refers to a non-toxic solid, semi-solid or liquid filler, diluent, encapsulating material or formulation auxiliary of any type.

Pharmaceutically acceptable carriers and excipient that may be used in the compositions of this invention include, but are not limited to, ion exchangers, alumina, aluminium stearate, lecithin, self-emulsifying drug delivery systems (SEDDS) such as d-a-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As appreciated by skilled artisans, the pharmaceutical composition is suitably formulated to be compatible with the intended route of administration. Examples of suitable routes of administration include topical route, oral route, intranasal route, sublingual route, intraocular route, parenteral route, including for instance intramuscular, subcutaneous, intravenous, intraperitoneal or local injections. The oral route can be used, provided that the composition is in a form suitable for oral administration, able to protect the active principle from the gastric and intestinal enzymes. Preferably the compound increasing endocannabinoids concentration, or the CB1 R agonist, or the TRPV1 agonist is administered by oral route, intranasal route or sublingual route.

Preferably, the pharmaceutical composition contains carriers that are pharmaceutically acceptable for an injectable formulation. They may in particular be sterile, isotonic, saline solutions (monosodium phosphate, disodium phosphate, sodium chloride, potassium chloride, calcium chloride or magnesium chloride etc., or mixtures of such salts), or dry, in particular lyophilized, compositions which by means of the addition, as appropriate, of sterilized water or physiological saline, can form injectable solutes.

As used herein, the term **"therapeutically effective amount"** means an amount of a compound or composition comprising said, that permits prevention and/or treatment of the subject social behavior disorder. The amount to be administered depends on the subject to be treated, on the ability of the subject's system to use the active ingredient, and on the desired degree of therapeutic effect. The precise amounts required for the administration depend on the choice of the physician and on the particularities of each individual. The amount naturally also depends on the active ingredient in question, on the mode of administration, and on the age and condition of the subject.

Preferably the compound increasing endocannabinoids concentration, or the CB1 R agonist, or the TRPV1 agonist is for use in improving social interactions.

The invention concerns the use of a compound increasing endocannabinoids concentration, or a CB1 R agonist, or a TRPV1 agonist for improving social interactions.

As used herein, the term **"improving social interactions"** means diminishing stress or apprehension before social encounters, decreasing irritability, improving facial emotion perception, improving emotional expression, improving social involvement, improving social cognition, increasing desire to form relationships and/or better ability to maintain relation ships.

Preferably the compound increasing endocannabinoids concentration in a male subject, or the CB1 R agonist, is for use for preventing and/or treating a social behavior disorder social in a male subject that had been exposed to cannabinoids *in utero.*

Preferably the use of a compound increasing endocannabinoid concentration or the use of a CB1 R agonist according to the invention is for the manufacture of a medicine for preventing and/or treating a social behavior disorder in a male subject that had been exposed to cannabinoids *in utero.*

Preferably the invention concerns a method for preventing and/or treating a social behavior disorder comprising administering to a male subject that had been exposed to cannabinoids *in utero* a therapeutically effective amount of a compound increasing endocannabinoids concentration or a therapeutically effective amount of a CB1 agonist.

Throughout the instant application, the term **"comprising"** is to be interpreted as encompassing all specifically mentioned features as well optional, additional, unspecified ones. As used herein, the use of the term "comprising" also discloses the embodiment wherein no features other than the specifically mentioned features are present (i.e. **"consisting of"**).

Furthermore the indefinite article "a" or "an" does not exclude a plurality.

The invention will be further illustrated in view of the following figures and examples.

### FIGURES:

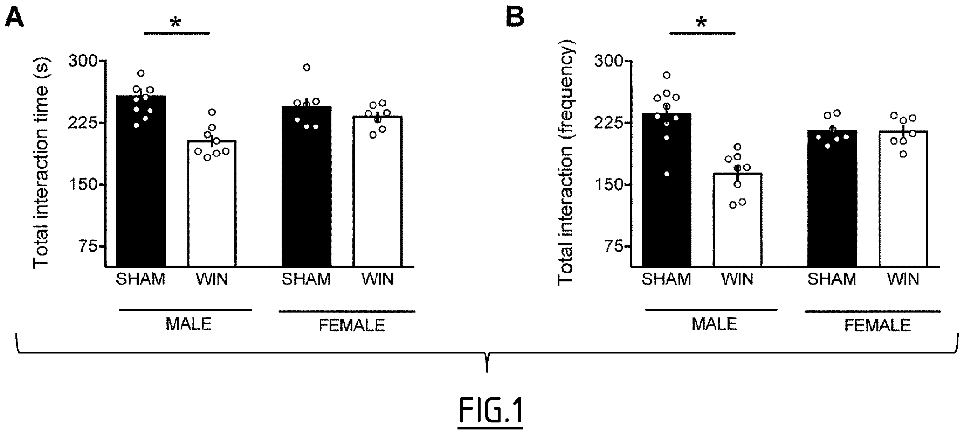

**Figure 1****. Sex specific modulation of social interaction in adult rats exposed *in utero* to the cannabinoid WIN55,212-2.**
   Adult male but not female rats exposed *in utero* to WIN55,212-2 spent less time to interact with a sex- and age-matched congener (A: F(sex x treat)1,28 = 7.09; p < 0.05, two-Way ANOVA) and had less contact with their congeners than male SHAM-animals (control animals) (B: F(sex x treat)1,28 = 15.54; p < 0.05, two-Way ANOVA). The impairment in sociability in adult male rats was not linked to impaired total motor locomotion, since there was no change in the distance travelled between *in utero* SHAM- or WIN-exposed male rats during the test (3758 ± 117.8 cm, n = 10; and 3779 ± 228.8 cm, n = 8; t(7) = 0.02, p > 0.05, t-test; SHAM- and WIN-exposed rats, respectively; data not shown). Scatter dot plot represents each animal. Error bars indicate SEM. *p < 0.05 Student-Newman-Keuls test.

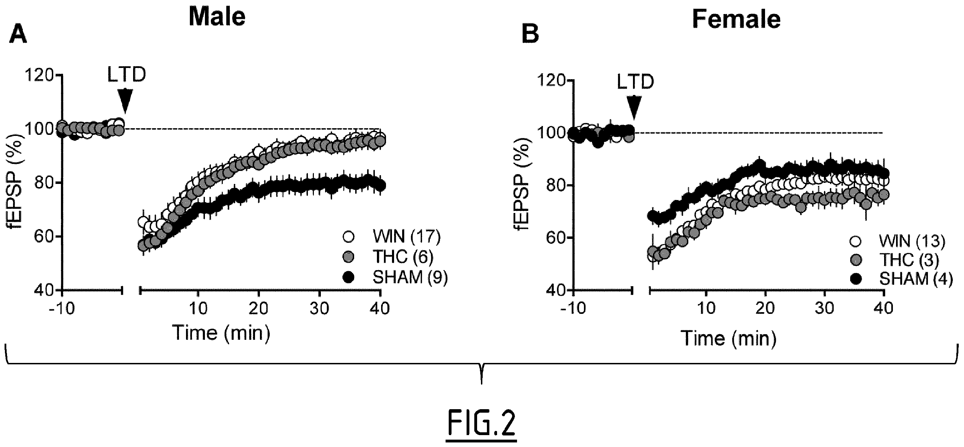

**Figure 2****. Sex specific ablation of LTD in the PFC of adult rats exposed *in utero* to WIN55,212-2 and THC (Tetrahydrocannabinol).**
   A. Average time courses of mean field EPSPs (excitatory postsynaptic potentials) showing that low-frequency stimulation (10 min at 10 Hertz, arrow) of excitatory inputs induced the LTD of evoked EPSPs recorded in the mPFC in SHAM (n = 9; black circles), but not in WIN (n = 17; white circles) or THC (n = 6; grey circles) *in utero*-exposed male rats. B. Average time courses of mean EPSPs showing that low-frequency stimulation induced LTD of evoked EPSPs in SHAM (n = 4; black circles), WIN (n = 13; white circles) and THC (n = 3; grey circles) *in utero*-exposed female rats. Error bars indicate SEM.
**Figure 3****. *In utero* exposure to WIN55,212-2 had no effect on LTD at accumbens in both male and female rats.**
   A. Average time courses of mean EPSPs showing that low-frequency stimulation induced LTD of evoked EPSPs recorded in SHAM (black circles) and WIN (white circles) *in utero*-exposed male (A) and female rats (B) (n = 4 - 5 animals per group). Error bars indicate SEM.
**Figure 4****. Sex specific mechanisms of LTD in the PFC of adult male and female rats: differential roles of CB1 R and TRPV1 receptors.**
   A. Average time courses of mean EPSPs showing that in Sham adult males but not in females rats (n = 6; white circles) LTD of evoked EPSPs required CB1 R receptor activation (n = 5; black circles), (mPFC slices preincubated for 45 min in 5 µM SR141716). B. Conversely, the specific TRPV1 antagonist capsazepine blocked LTD of evoked EPSPs recorded in adult Sham-female (n = 3; white circles), but not male (n = 4; black circles) (mPFC slices preincubated for 45 min in 10 µM capsazepine). C-D. Average time courses of mean EPSPs showing that in *in-utero* WIN55,212-2 exposed adult females, LTD of evoked EPSPs is not mediated by CB1 R receptors (C) but rather by TRPV1 (D) (n = 5; white circles for both conditions) (mPFC slices preincubated for 45 min in 5 µM SR141716 (C) and 10 µM capsazepine (D). Error bars indicate SEM.

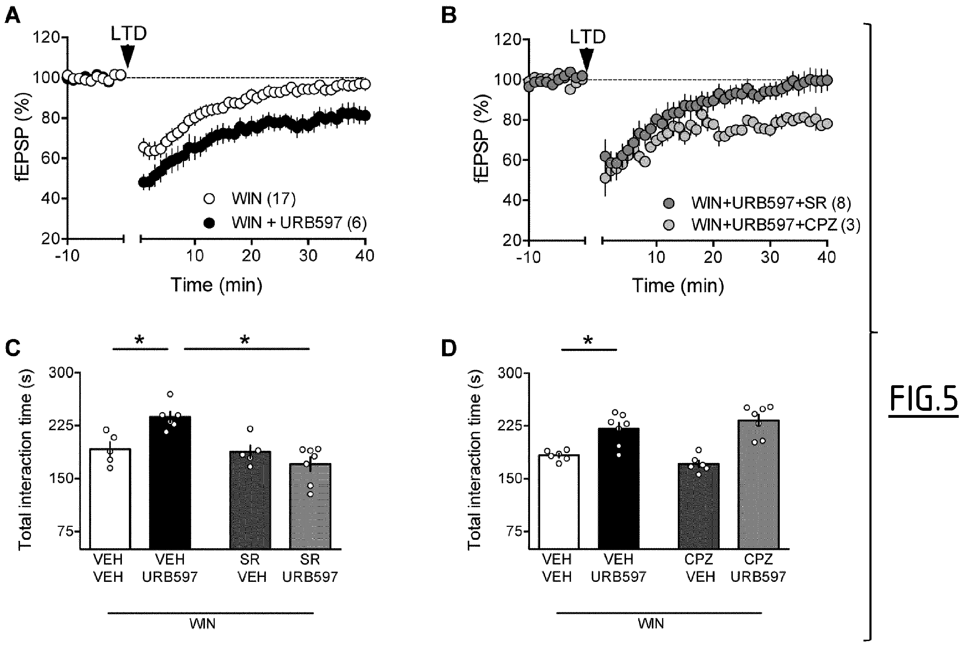

**Figure 5****. Enhancing anandamide by URB597 restores normal LTD and social interaction in *in-utero* exposed male rats, via CB1 R and not TRPV1 receptors.**
   A. Time course of normalized fEPSP responses from mPFC slices preincubated with 2 µM URB597 (n = 6; black circles) in adult male rats exposed *in utero* to WIN55,212-2 (n = 17; white circles). B. Average time courses of mean EPSPs showing that URB597 rescued from LTD deficits in adult male rats via CB1 R (n = 8; dark grey circles) (mPFC slices preincubated for 45 min in 5 µM SR141716, SR) and not TRPV1 activation (n = 3; grey circles) (mPFC slices preincubated for 45 min in 10 µM capsazepine, CPZ). C. Pre-treatment with the CB1R antagonist SR141716 (1 mg/kg, i.p.) prevented the curating actions of URB597 (0.1 mg/Kg, i.p.) on social interaction (F(URB x SR)1,19 = 11.46; p < 0.05, two-Way ANOVA). "VEH" corresponds to treatment with only the vehicle thus corresponding to controls. D. In contrast, the TRPV1 antagonist capsazepine did not block the curating effects of URB597 on social interaction in adult rats exposed to WIN55,212-2 *in utero* (F(URB x CPZ)1,22 = 3.076; p > 0.05, two-Way ANOVA). Scatter dot plot represents each animal. Error bars indicate SEM. *p < 0.05 Student-Newman-Keuls test.

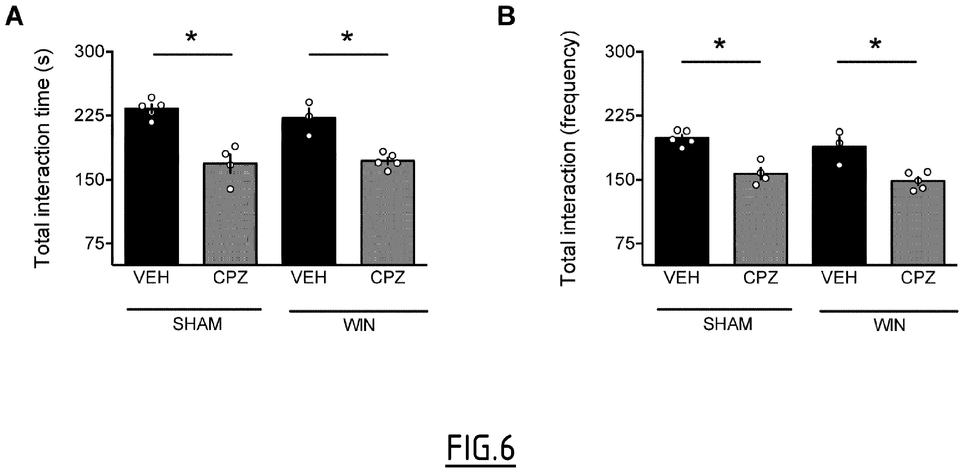

**Figure 6****. Blocking TRPV1 receptors in female rats exposed *in utero* to WIN55,212-2 reduces social interaction.**
   The TRPV1 antagonist capsazepine (5 mg/Kg; i.p.) reduced the total time (A: F(sex x treat)1,13 = 0.885; p > 0.05, two-Way ANOVA) and frequency (B: F(sex x treat)1,13 = 0.02; p > 0.05, two-Way ANOVA) of social interaction in both adult female rats exposed to WIN55,212-2 or in control solution *in utero.* Scatter dot plot represents each animal. Error bars indicate SEM.

### Examples :

### Materials and methods

### A) Animals

Wistar female rats (Charles River, France) weighing 250-280 g were housed at constant room temperature (20 ± 1°C) and humidity (60%), and exposed to a light cycle of 12 h/day (08:00 a.m. to 08:00 p.m.), with food and water available ad libitum. For mating, pairs of females were placed with single male rats in the late afternoon. Vaginal smears were taken the following morning at 09:00 a.m. The day on which sperm was present was designated as the day 0 of gestation (GD 0).

Treatment was performed daily with the CB1 R receptor agonist WIN55,212-2 (0.5 mg/kg) or Delta9-Tetrahydrocannabinol (THC, 5 mg/kg) or their respective vehicles from GD 5 to GD 20. WIN55,212-2 was suspended in 5% DMSO, 5% cremophor and saline, and injected s.c. at a volume of 1 ml/kg. Control dams (Sham group) received a similar volume injection of vehicle solution. THC was suspended in 5% Ethanol, 5% cremophor and saline, and injected s.c. at a volume of 1 ml/kg.

Pups were weaned at 21 days of age. For behavioral and electrophysiological experiments male and female rats were tested at adulthood (postnatal day 90-120).

### B) Behavior

All animals were experimentally naïve and used only once.

### SOCIAL INTERACTION

The test was performed in a sound attenuated chamber under dim light conditions. The testing arena consisted of a Plexiglas cage measuring 45 × 45 cm, with 2 cm of wood shavings covering the floor. Social behavior was assessed as previously described (Trezza et al., 2012).

Briefly, adult rats were individually habituated to the test cage for 5 min (adult rats) on each of the 2 days prior to testing. Before testing, adult animals were socially isolated for 24 h, respectively, to enhance their social motivation and thus facilitate the expression of social behaviors during testing. Drug treatments were counterbalanced so that cage mates were allocated to different treatment groups. The animals of each pair were similarly treated, did not differ more than 10 g in body weight and were not cage mates. Behavior was assessed per pair of animals and analyzed by a trained observer who was unaware of treatment condition using the Ethovision XT video tracking software (Noldus, Wageningen, The Netherlands).

The test consisted of placing two similarly treated animals into the test cage for 10 min (adult rats). In rats, a bout of social play behavior starts with one rat soliciting ('pouncing') another animal, by attempting to nose or rub the nape of its neck. The animal that is pounced upon can respond in different ways. If the animal that is pounced upon fully rotates to its dorsal surface, 'pinning' is the result, i.e., one animal lying with its dorsal surface on the floor with the other animal standing over it. From this position, the supine animal can initiate another play bout, by trying to gain access to the other animal's neck. Thus, during social play, pouncing is considered an index of play solicitation, while pinning can be regarded as the terminal component of a single play bout as well as a releaser of a prolonged play bout. Pinning and pouncing frequencies can be easily quantified and they are considered to be the most characteristic parameters of social play behavior in rats. During the social encounter, animals may also display social behaviors not directly associated with play, such as sniffing or grooming the partner's body. A pair of rats was considered as one experimental unit.

In adult rats, the total time and total frequency of active social interactions were obtained as the sum of the time and frequency of the following behavioral elements scored per 10 min:
- Play-related behaviors: pouncing, pinning and boxing.
- Social behaviors unrelated to play: social exploration (sniffing any part of the body of the test partner), social grooming (one rat licks and chews the fur of the conspecific, while placing its forepaws on the back or the neck of the other rat), following/chasing (walking or running in the direction of the partner which stays where it is or moves away), crawling under/over (one animal crawls underneath or over the partner's body, crossing it transversely from one side to the other), kicking (the rat kicks backwards at the conspecific with one or both hind paws).

### DRUG TREATMENT

The fatty acid amide hydrolase (FAAH) inhibitor URB597 [(3'-(aminocarbonyl)[1,1'-biphenyl]-3-yl)-cyclohexylcarbamate] (Tocris), the CB1 R cannabinoid receptor antagonist SR141716 [5-(4-chloro-phenyl)-1-(2,4-dichlorophenyl)-4-methyl-N-1-piperidinyl-1H-pyrazole-3-carboxamide] (National Institute of Mental Health, USA) and the vanilloid TRPV1 antagonist capsazepine (N-[2-(4-Chlorophenyl)ethyl]-1,3,4,5-tetrahydro-7,8-dihydroxy-2H-2-benzazepine-2-carbothioamide) (Tocris) were dissolved in 5% Tween 80/5% polyethylene glycol/saline and given intraperitoneally (i.p.).

URB597 (0.1 mg/kg) or its vehicle was administered 2 h before testing; SR141716 was administered 30 min before URB597, at a dose that did not induce effects by itself (1 mg/kg) in adult rats. Capsazepine (5 mg/kg) was injected 30 min before testing. Drug doses and pre-treatment intervals were based on the literature (Ratano et al., 2017) and on pilot experiments. Solutions were freshly prepared on the day of the experiment and were administered in a volume of 1 ml/kg in adult rats.

### STATISTICAL ANALYSIS

All the behavioral parameters were expressed as mean ± SEM. Group comparisons used t-test and two-way ANOVA (for different sex and pharmacological treatments), followed by Student-Newman-Keuls post hoc tests where appropriate.

### C) Physiology

### SLICE PREPARATION

Adult male and female rats were anesthetized with isoflurane and killed as previously described (Martin and Manzoni, 2014). The brain was sliced (300 µm) in the coronal plane with a vibratome (Integraslice, Campden Instruments) in a sucrose-based solution at 4°C (in mm as follows: 87 NaCl, 75 sucrose, 25 glucose, 2.5 KCI, 4 MgCl2, 0.5 CaCl2, 23 NaHCO3 and 1.25 NaH2PO4). Immediately after cutting, slices containing the medial prefrontal cortex (PFC) or accumbens were stored for 1 h at 32°C in a low calcium ACSF that contained (in mm) as follows: 130 NaCl, 11 glucose, 2.5 KCI, 2.4 MgCl2, 1.2 CaCl2, 23 NaHCO3, 1.2 NaH2PO4, and were equilibrated with 95% 02/5% CO2 and then at room temperature until the time of recording.

### ELECTROPHYSIOLOGY

Field potential recordings were made in coronal slices containing the mPFC or the accumbens as previously described (Robbe et al., 2002). During the recording, slices were placed in the recording chamber and superfused at 2 ml/min with either low Ca2+ ACSF for mPFC or normal ACSF for the accumbens. All experiments were done at 32°C or room temperature for mPFC and accumbens, respectively. The superfusion medium contained picrotoxin (100 µM) to block GABA Type A (GABA-A) receptors. All drugs were added at the final concentration to the superfusion medium.

For extracellular field experiments, the recording pipette was filled with ACSF. The glutamatergic nature of the field EPSP (fEPSP) was systematically confirmed at the end of the experiments using the ionotropic glutamate receptor antagonist CNQX (20 µM), which specifically blocked the synaptic component without altering the nonsynaptic component (data not shown).

Both fEPSP area and amplitude were analyzed. Stimulation was performed with a glass electrode filled with ACSF and the stimulus intensity was adjusted ∼60% of maximal intensity after performing an input-output curve (baseline EPSC amplitudes ranged between 50 and 150 pA). Stimulation frequency was set at 0.1 Hz.

### DATA ACQUISITION AND ANALYSIS

The magnitude of plasticity was calculated 38-40 min after induction as percentage of baseline responses. Statistical analysis of data was performed with Prism (GraphPad Software) using tests indicated in the main text after outlier subtraction. All values are given as mean ± SEM, and statistical significance was set at p < 0.05.

### Results

### Sex specific modulation of social interaction and LTD in adult rats exposed in utero to the cannabinoids WIN55,212-2 and THC.

We found that *in utero* exposure to the cannabinoid WIN55,212-2 sex-specifically modified social behavior at adulthood. We observed that adult male rats spent less time in social interactions (Fig. 1 A) and had less contact with their congeners than male rats exposed *in utero* to the control solution. The increased social anxiety-like behavior was not linked to impaired total motor locomotion, and there was no significant change in the distance traveled between WIN55,212-2 and Sham groups during testing (data not shown). These effects appeared male specific since adult female rats showed a normal behavioral profile with no difference in the total time (Fig. 1 A) and total frequency (Fig. 1 B) of social interaction in both *in utero* WIN55,212-2 and Sham exposed groups.

In view of this result showing a reduction in the social interaction, a prefrontal cortex modulated behavior, we decided to interrogate a critical actor of plasticity and reliable prefrontal endophenotypes of neuropsychiatric diseases: the endocannabinoid/mGlu5-dependent LTD.

We found that, in mPFC slices prepared from adult male rats exposed *in utero* to Sham solution, tetanic stimulation induced a robust, endocannabinoid mediated LTD (eCB-LTD) of excitatory synapses onto layer 5 mPFC pyramidal neurons. This eCB-LTD was absent in adult male rats exposed *in utero* to the cannabinoid WIN55,212-2 and THC (Fig. 2A). Interestingly, in adult female rats eCB-LTD was indistinguishable in all the treatment groups (Fig. 2B) highlighting a sex specific ablation of LTD in the mPFC of adult rats exposed *in utero* to WIN55,212-2 and THC.

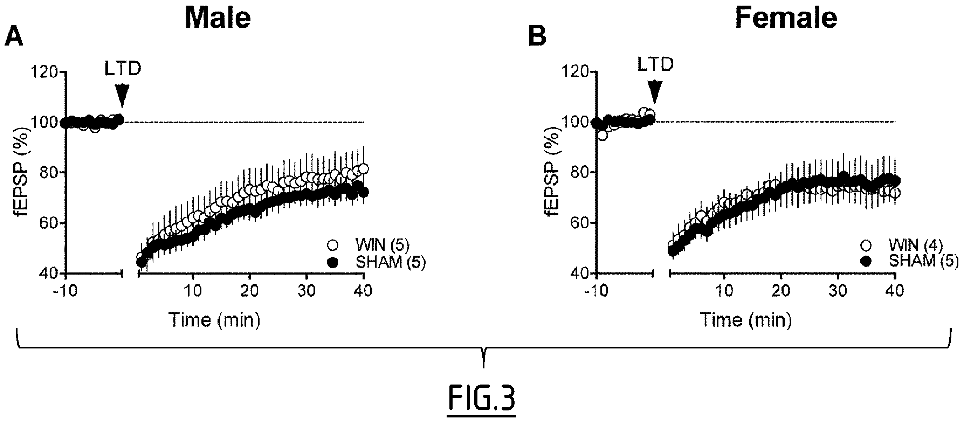

We recently showed that eCB-mediated plasticity in the accumbens is instrumental in underlying depression-like and emotional behavior. Therefore, we decided to test whether *in utero* exposure to the cannabinoid WIN55,212-2 could impact accumbens synapses and synaptic plasticity at adulthood. In accumbens slices prepared from adult male and female rats exposed *in utero* to WIN55,212-2, tetanic stimulation induced a robust eCB-LTD of excitatory synapses onto medium spiny neurons (MSNs) in both male (Fig. 3A) and female (Fig. 3B) adult rats, which matched that observed in adult male and female rats exposed *in utero* to Sham solution. These data show that *in utero* exposure to the cannabinoid WIN55,212-2 is sufficient to selectively abolish the development of mGlu5-eCB-mediated synaptic plasticity in the adult rat PFC but not accumbens.

### Sex specific mechanisms of LTD in the PFC of adult male and female rats: differential roles of CB1 R and TRPV1 receptors.

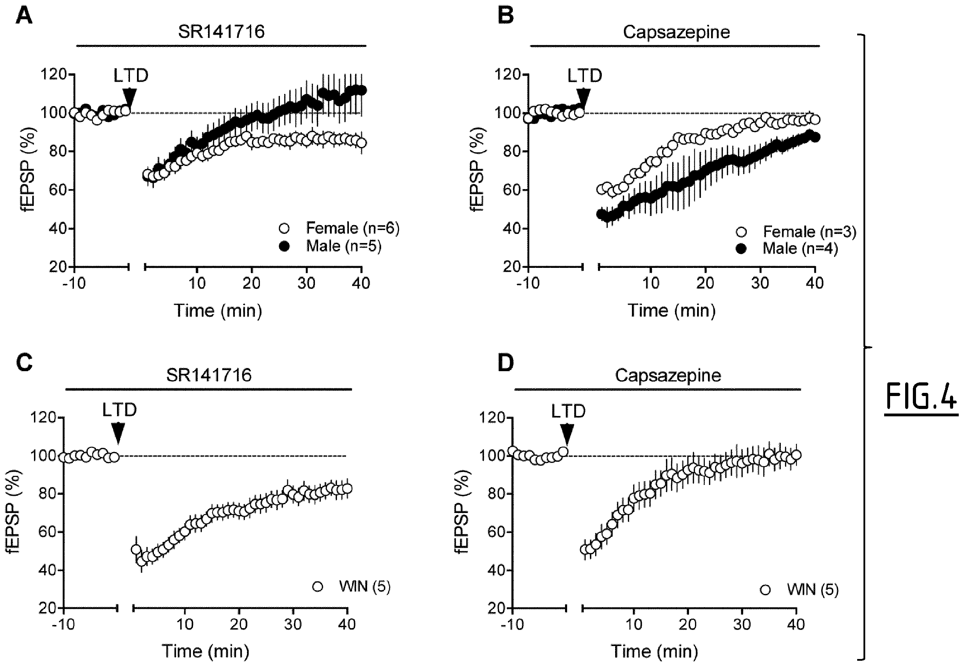

To identify a possible mechanism underlying sex specific alterations of the adult rat PFC synaptic plasticity, we verified that eCB-LTD of excitatory mPFC synapses in Sham animals was mediated by CB1 R receptors; preincubation with the CB1 R antagonist SR141716 completely blocked LTD in adult male but not female rats (Fig. 4A). Conversely, preincubation with the selective vanilloid TRPV1 antagonist capsazepine completely blocked LTD in adult Sham female but not male rats (Fig. 4B). These TRPV1 mechanism in Sham adult female was also confirmed in adult female exposed *in utero* to the synthetic cannabinoid WIN55,212-2 (Fig. 4C and D).

### Enhancing anandamide by URB597 restores normal LTD and social interaction in in-utero exposed male rats, via CB1 R and not TRPV1 receptors.

Pharmacological exploration of synaptic plasticity deficits in the brain slice preparation is a valuable approach to identify new therapeutic targets.

The lack of eCB-mediated LTD in the mPFC of adult male rats exposed *in utero* to WIN55,212-2 prompted us to test the new hypothesis that amplification of some of its key molecular elements could restore synaptic plasticity. Therefore, we tested whether inhibition of anandamide (AEA)'s main degrading enzyme fatty acid hydrolase (FAAH) would allow AEA levels to reach the threshold for LTD induction in WIN55,212-2 *in utero* exposed male rats (Fig. 5A). PFC slices were incubated with the FAAH inhibitor URB597 (2 µM) for 45-90 min before LTD induction. This treatment was sufficient to restore eCB-LTD in the PFC of adult male rats exposed *in utero* to WIN55,212-2 (Fig. 5A). These effects were mediated by CB1 R but not TRPV1 activation since the CB1 R antagonist SR141716 (5 µM) but not the selective TRPV1 antagonist capsazepine (10 µM) blocked the URB597 rescue, showing that specifically CB1 R activation mediates the effects of the FAAH inhibitor in the PFC (Fig. 5B).

We next tested whether pharmacological enhancements of the eCB signaling could normalize social behavioral deficits. Acute pharmacological enhancement of AEA with URB597 (0.1 mg/kg, i.p.) ameliorated social deficits in adult male rats exposed *in utero* to WIN55,212-2. Social interaction was normalized (Fig. 5C and D), suggesting that the rescue effects of URB597 extended to the social domain.

Of note, pre-treatment with the CB1R antagonist SR141716 (1 mg/kg, i.p.) prevented the curating actions of URB597 (0.1 mg/Kg, i.p.; Fig. 5C) on social interaction. In contrast, the TRPV1 antagonist capsazepine did not block the curating effects of URB597 on social interaction in adult rats exposed to WIN55,212-2 *in utero* (Fig. 5D).

Thus, blocking AEA's degradation normalized social deficits induced by in utero exposure to WIN55,212-2 in adult male rats via CB1 R and not TRPV1 activation.

### Blocking TRPV1 receptors in female rats exposed in utero to WIN55,212-2 reduces social interaction.

The TRPV1 antagonist capsazepine (5 mg/Kg; i.p.) reduced the total time (Fig. 6A) and frequency (Fig. 6B) of social interaction in Sham adult female rats confirming the involvement of TRPV1 rather than CB1 R receptors in the modulation of social behavior at adulthood in female rats. These effects were also replicated in adult female rats exposed to WIN55,212-2 *in utero* (Fig. 6A and B).

### Bibliography

Lebovitz EE, Keller JM, Kominsky H, Kaszas K, Maric D, ladarola MJ (2012) Positive allosteric modulation of TRPV1 as a novel analgesic mechanism. Molecular Pain 8:70.
Martin HG, Manzoni OJ (2014) Late onset deficits in synaptic plasticity in the valproic acid rat model of autism. Front Cell Neurosci 8:23.
Ratano P, Palmery M, Trezza V, Campolongo P (2017) Cannabinoid Modulation of Memory Consolidation in Rats: Beyond the Role of Cannabinoid Receptor Subtype 1. Front Pharmacol 8:200.
Robbe D, Kopf M, Remaury A, Bockaert J, Manzoni OJ (2002) Endogenous cannabinoids mediate long-term synaptic depression in the nucleus accumbens. Proc Natl Acad Sci U S A 99:8384-8388.
Scott EC, Kendall DA (2017) Assessing allosteric modulation of CB1 at the receptor and cellular levels. Methods in enzymology 593(14): 317-342.
Trezza V, Damsteegt R, Manduca A, Petrosino S, Van Kerkhof LW, Pasterkamp RJ, Zhou Y, Campolongo P, Cuomo V, Di Marzo V, Vanderschuren LJ (2012) Endocannabinoids in amygdala and nucleus accumbens mediate social play reward in adolescent rats. J Neurosci 32(43):14899-908.

## Claims

1. A compound increasing endocannabinoid concentration in a subject for use for preventing and/or treating a social behavior disorder.

2. The compound for the use according to claim 1, wherein said compound inhibits a degrading enzyme of endocannabinoids.

3. The compound for the use according to claim 1 or 2, wherein the endocannabinoid which concentration is increased is anandamide and/or 2-AG (2-Arachidonoylglycerol).

4. A CB1R agonist for the use for preventing and/or treating a social behavior disorder social in a male subject.

5. The CB1 R agonist for the use according to claim 4, wherein said agonist is a Dronabinol, Epigallocatechin, Epicatechin, Kavain, Yangonin, N-Arachidonoyl dopamine, Cannabinol, HU-210, 11-Hydroxy-THC, Levonantradol, 2-Arachidonyl glyceryl ether, JWH-073, Tetrahydrocannabinol, 2-Arachidonoylglycerol, AM-2201, CP 55,940, JWH-018, WIN 55,212-2 or GAT228 or pharmaceutically acceptable salts thereof.

6. The CB1 R agonist for the use according to claim 4, wherein said agonist is a positive allosteric modulator, and preferably is lipoxin A4, GAT211, ZCZ011, RTI-370, RTI-371, JHW007 or an analogue thereof.

7. A TRPV1 agonist for use for preventing and/or treating a social behaviors disorder in a female subject.

8. The TRPV1 agonist for the use according to claim 7, wherein said agonist is a lipid vanilloid, or pharmaceutically acceptable salts thereof.

9. The TRPV1 agonist for the use according to claim 7, wherein said agonist is a positive allosteric modulator, and preferably is MRS1477 or a MRS1477 analogue.

10. The compound for the use according to any one of claims 1-3, or the CB1 R agonist for the use according to any one of claims 4-6, or the TRPV1 agonist for the use according to any one of claims 7-9, wherein said compound or agonist is administered by oral route, intranasal route or sublingual route.

11. The compound for the use according to any one of claims 1-3, or the CB1 R agonist for use according to any one of claims 4-6, or the TRPV1 agonist for use according to any one of claims 7-9, wherein the social behaviors disorder is autism, excessive shyness, sociopathy, schizophrenia, bipolar disorder, irritability, addiction tendencies, anxiety, mood disorder or depression such as major depressive illness.

12. The compound for the use according to any one of claims 1-3, or the CB1 R agonist for use according to any one of claims 4-6, or the TRPV1 agonist for use according to any one of claims 7-9, for use in improving social interactions.

13. The compound for the use according to any one of claims 1-3 or the CB1 R agonist for use according to any one of claims 4-6, wherein the subject is a male that had been exposed to cannabinoids *in utero.*
